(19) Europäisches Patentamt
European Patent Office
Office européen des brevets

(11) Numéro de publication: **0 252 809**
**A2**

(12) # DEMANDE DE BREVET EUROPEEN

(21) Numéro de dépôt: **87401522.5**

(22) Date de dépôt: **01.07.87**

(51) Int. Cl.4: **C 07 D 471/04**
C 07 D 513/04,
C 07 D 487/04,
C 07 D 495/04,
A 61 K 31/435,
A 61 K 31/425,
A 61 K 31/495, A 61 K 31/40,
A 61 K 31/505,
A 61 K 31/415, C 07 D 235/24

(30) Priorité: **08.07.86 FR 8609886**

(43) Date de publication de la demande:
**13.01.88 Bulletin 88/02**

(84) Etats contractants désignés:
**AT BE CH DE ES FR GB GR IT LI LU NL SE**

(71) Demandeur: **SYNTHELABO**
**58 rue de la Glacière**
**F-75013 Paris (FR)**

(72) Inventeur: **Manoury, Philippe**
**L'Orée de Verrières 38, Avenue des Vaupépins**
**F-91370 Verrières Le Buisson (FR)**

**Binet, Jean**
**12, Hameau de la Gondole**
**F-91650 Breuillet (FR)**

**Aletru, Michel**
**40 à 52, rue des Grands Champs**
**F-75020 Paris (FR)**

(74) Mandataire: **Thouret-Lemaitre, Elisabeth et al**
**Service Brevets - SYNTHELABO 58, rue de la Glacière**
**F-75621 Paris Cédex 13 (FR)**

(54) **Dérivés du nitrofuranne, leur préparation et leur application en thérapeutique.**

(57) Dérivés du nitrofuranne, répondant à la formule (I)

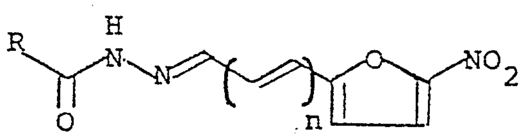

(I)

dans laquelle n est 0 ou 1 et R est un radical hétérocyclique.
Application en thérapeutique.

EP 0 252 809 A2

**Description**

DERIVES DU NITROFURANNE, LEUR PREPARATION ET LEUR APPLICATION EN THERAPEUTIQUE

La présente invention a pour objet des dérivés du nitrofuranne, leur préparation et leur application en thérapeutique.

Les composés de l'invention répondent à la formule générale (I) donnée en annexe dans laquelle n est 0 ou 1 et R est un radical imidazo[1,2-a]pyridinyle-2, imidazo[2,1-b]thiazolyle-6, imidazo[1,2-a]pyrazinyle-2, tétrahydro-5,6,7,8 imidazo[1,2-a]pyrazinyle-2, acétyl-7 tétrahydro-5,6,7,8 imidazo[1,2-a]pyrazinyle-2, imidazo[1,5-a]pyridinyle-1, imidazo[1,5-a]pyridinyle-3, pyrazolo[1,5-a]pyridinyle-3, indolizinyle-1, indolizinyle-3, tétrahydro-5,6,7,8 indolizinyle-1, tétrahydro-5,6,7,8 indolizinyle-2, 6H-thieno[2,3-b]pyrrolyle-5, 4H-thiéno[3,2-b]pyrrolyle-5, oxo-4 4H-pyrido[1,2-a]pyrimidinyle-3, 2H-benzimidazolyle-2, imidazo[1,2-a]pyridinyle-3.

Selon l'invention, on peut préparer les composés (I) selon le schéma réactionnel de l'annexe par condensation entre l'hydrazide correspondant (II) et le nitro-5 furannecarboodéhyde-2 (ou son vinylogue) dans un solvant alcoolique (comme le méthanol, l'éthanol ou le méthoxy-2 éthanol) maintenu entre 20°C et sa température de reflux. Les dérivés obtenus cristallisent spontanément dans le milieu réactionnel.

Le (nitro-5 furannyl-2)-3 propène-2 al est préparé par une réaction d'aldolisation entre le nitro-5 furannecarboxaldéhyde-2 et l'acétaldéhyde, suivie d'une purification par chromatographie sur colonne de silice.

Les hydrazides (II) peuvent être préparés par réaction entre les acides ou les esters des acides correspondants décrits dans la littérature et l'hydrazine sèche en présence ou non d'agents de condensation (comme par exemple le dicyclohexylcarbodiimide, l'éthoxy-2 éthoxycarbonyl-1 dihydro-1,2 quinoléine, etc).

Les exemples suivants illustrent l'invention.

Les analyses et les spectres IR et RMN confirment la structure des composés.

Exemple 1. [(Nitro-5 furannyl-2)méthylène]hydrazide de l'acide imidazo[1,2-a]pyridinecarboxylique-2.

1.1. Hydrazide de l'acide imidazo[1,2-a]pyridinecarboxylique-2.

On ajoute 4,2 ml de triéthylamine à une suspension de 8,1 g de bromhydrate d'imidazo[1,2-a]pyridinecarboxylate-2 d'éthyle (décrit par J.G. Lombardino, J. Org. Chem. 30, 2403, 1965) dans 75 ml de toluène. Après avoir filtré et évaporé le toluène, on reprend le résidu avec 75 ml d'éthanol et 6 ml d'hydrazine puis porte le mélange à la température du reflux pendant 4 h.

On filtre le précipité et obtient l'hydrazide de l'acide imidazo[1,2-a] pyridinecarboxylique-2.
F = 195-198°C.

1.2. [(Nitro-5 furannyl-2)méthylène]hydrazide de l'acide imidazo[1,2-a]pyridinecarboxylique-2.

On porte à la température du reflux pendant 3 h une suspension de 5,2 g (0,037 mole) de nitro-5 furannecarboxaldéhyde-2 et de 6 g (0,034 mole) de l'hydrazide précédemment obtenu dans 150 ml de méthanol.

Après refroidissement, on filtre le précipité, on le reprend avec du méthanol bouillant, on le filtre et le sèche. On obtient le composé.
F = 280°C.

Exemple 2. [(nitro-5 furannyl-2) propène-2 ylidène-1] hydrazide de l'acide imidazo[2,1-b]thiazolecarboxylique-6.

Selon le même mode opératoire que dans l'exemple 1.2., on fait réagir 1,1 g (0,065 mole) de (nitro-5 furannyl-2)-3 propène-2al et 1,2 g d'hydrazide de l'acide imidazo[2,1-b] thiazolecarboxylique-6 dans du méthanol. On obtient le composé qui fond à 332°C.

Exemple 3 [(nitro-5 furannyl-2)métylène]hydrazide de l'acide acétyl-7 tétrahydro-5,6,7,8 imidazo[1,2-a]pyrazine-carboxylique-2.

3.1. Hydrazide de l'acide acétyl-7 tétrahydro-5,6,7,8 imidazo [1,2-a]pyrazinecarboxylique-2.

On ajoute 5,9 g (0,03 mole) de tétrahydro-5,6,7,8 imidazo [1,2-a]pyrazinecarboxylate-2 d'éthyle (décrit par J.P. Chapat, J. Chem. Research, 1984, 468-80), à 8,3 ml d'anhydride acétique refroidi à 0-5°C et abandonne le mélange 2 jours.

On verse celui-ci sur de l'eau glacée, extrait avec du chlorure de méthylène puis lave la phase organique avec de l'eau jusqu'à pH neutre. On sèche la phase organique, la filtre et l'évapore. On obtient l'acétyl-7 tétrahydro-5,6,7,8 imidazo [1,2-a]pyrazine-carboxylate-2 d'éthyle. F = 97°C.

On porte à la température du reflux, pendant 20 mn, 6 g de l'ester ainsi obtenu dans 10 ml d'hydrazine. On évapore l'hydrazine en excès et purifie l'huile obtenue par chromatographie sur une colonne de silice (chlorure de méthylène, méthanol, ammoniaque 19/10/1).

On obtient l'hydrazide.
F = 182°C.

3.2. [(Nitro-5 furannyl-2)méthylène]hydrazide de l'acide acétyl-7 tétrahydro-5,6,7,8 imidazo [1,2-a]pyrazine-carboxylique-2.

Selon le même mode opératoire que dans l'exemple 1.2., on fait réagir 1,9 g de l'hydrazide obtenu précédemment et 1,3 g de nitro-5 furannecarboxaldéhyde-2 dans du méthanol. On obtient le composé qui fond à 255° C.

Exemple 4. [(Nitro-5 furannyl-2)méthylène]hydrazide de l'acide phényl-3 imidazo[1,2-a]pyridinecarboxylique-2.

4.1. Bromo-3 phényl-3 oxo-2 propionate d'éthyle.

A une solution de 13,9 g (0,072 mole) de oxo-2 phényl-3 propanoate d'éthyle (décrit par E.L. Eliel et coll. J. Org. Chem. 37, 505, 1972) dans 250 ml de tétrahydrofuranne (THF) et 6,1 g (0,072 mole) de pyrrolidinone-2, on introduit, à 10° C environ, par portions, 39,3 g (0,08 mole) de hydrotribromure de pyrrolidone (PHT). On laisse la température du mélange revenir à 20° C, puis chauffe à 50° C pendant 1 h. On refroidit le mélange puis le verse sur de la glace. On extrait avec de l'éther, lave la phase organique avec de l'eau, sèche avec $MgSO_4$, filtre et évapore. On obtient 18 g d'huile que l'on utilise brute pour la suite de la réaction.

4.2. Phényl-3 imidazo[1,2-a]pyridinecarboxylate-2 d'éthyle.

On ajoute lentement, à la température ambiante, 19,5 g (0,07 mole) de l'huile précédente en solution dans 70 ml de diméthoxyéthane à une solution de 13,4 g (0,143 mole) d'amino-2 pyridine dans 100 ml de diméthoxyéthane. Lorsque l'addition est terminée on chauffe à 80-90° C pendant 2 h puis évapore. On reprend le résidu d'évaporation avec de l'eau, extrait avec du chlorure de méthylène, on sèche avec $MgSO_4$, filtre, évapore. On purifie le produit brut par chromatographie sur colonne de silice (éluant = $CH_2$ $Cl_2$ -$CH_3$ OH 95.5).

On obtient 6,2 g de phényl-3 imidazo[1,2-a] pyridinecarboxylate-2 d'éthyle. F = 106° C.

On obtient de la même façon le phényl-2 imidazo[1,2-a]pyridinecarboxylate-3 d'éthyle (F = 68° C) et l'(hydroxy-4 phényl)-2 imidazo[1,2-a]pyridinecarboxylate-3 d'éthyle F = 220° C).

4.3. Hydrazide de l'acide phényl-3 imidazo[1,2-a]pyridinecarboxylique-2.

On prépare l'hydrazide comme décrit dans l'exemple 1.1. en faisant réagir 5,6 g (0,02 mole) de l'ester précédent avec 4 ml d'hydrazine anhydre dans 150 ml d'éthanol. On obtient le composé qui fond à 233° C.

4.4. [(Nitro-5 furannyl-2) méthylène]hydrazide de l'acide phényl-3 imidazo[1,2-a]pyridinecarboxylique-2.

En utilisant le mode opératoire décrit en 1.2., on fait réagir 2,5 g (0,01 mole) de l'hydrazide précédent, 1,6 g (0,011 mole) de nitro-5 furannecarboxaldéhyde-2 dans 100 ml de méthanol.

On obtient le produit qui fond à 245° C.

Exemple 5. [(Nitro-5 furannyl-2)méthylène]hydrazide de l'acide méthylsulfinyl-3 imidazo[1,5-a]pyridinecarboxylique-1.

5.1. Méthylsulfinyl-3 trifluoroacétyl-1 imidazo[1,5-a] pyridine.

On refroidit à 5° C une solution de 15,6 g (0,06 mole) de méthylthio-3 trifluoroacétyl-1 imidazo[1,5-a]pyridine (J. Bourdais et coll. J. Het. Chem. 17, 1351, 1980) dans 750 ml de chloroforme. On ajoute en 1 h environ 30,6 g (0,132 mole) d'acide m-chloroperbenzoïque.

On agite 1 h 30, filtre le précipité, lave la phase organique avec une solution de bisulfite de sodium, puis une solution de bicarbonate de sodium, puis avec de l'eau. On sèche le chloroforme avec $MgSO_4$, filtre, évapore. On purifie le produit par chromatographie sur une colonne de silice. On obtient le produit qui fond à 140° C.

5.2. Acide méthylsulfinyl-3 imidazo[1,5-a]pyridine carboxylique-1.

On ajoute, par portions, à 5° C environ, 15,3 g (0,05 mole) du produit précédent à 200 ml d'éthanol à 87,5 % contenant 59 g de potasse. On agite 20 mn après la fin de l'addition, puis filtre le précipité obtenu. On reprend celui-ci dans 100 ml d'eau, acidifie à pH 1 avec HCl 3 N, agite 1 h, filtre le précipité et le sèche. On obtient le produit qui fond à 200° C.

5.3. Hydrazide de l'acide méthylsulfinyl-3 imidazo[1,5-a] pyridinecarboxylique-1.

On ajoute 2 ml de $Et_3$ N à 3 g (0,013 mole) de l'acide précédent en solution dans 100 ml de chloroforme. On refroidit la solution à 0° C environ et ajoute 1,4 ml (0,015 mole) de chloroformiate d'éthyle en solution dans 5 ml de chloroforme. On agite la solution 1 h 30 à 5° C puis ajoute celle-ci à 130 ml de chloroforme contenant 4,2 ml d'hydrazine.

On laisse la température revenir à 20° C, agite 1 h 30, filtre le précipité. On obtient ainsi le produit qui fond à 188° C.

5.4. [(Nitro-5 furannyl-2)méthylène]hydrazide de l'acide méthylsulfinyl-3 imidazo[1,5-a]pyridinecarboxylique-1.

Ce produit est préparé en utilisant la méthode décrite dans l'exemple 1 à partir de 1,5 g de l'hydrazide précédent dans 300 ml de méthanol et de 1 g (0,006 mole) de nitro-5 furannecarboxaldéhyde-2. F = 294° C.

Exemple 6. [(Nitro-5 furannyl-2)méthylène]hydrazide de l'acide imidazo[1,5-a]pyridinecarboxylique-1.

6.1. Imidazo[1,5-a]pyridinecarboxylate-1 d'éthyle.

On porte au reflux, 16 h, 11,2 g (0,078 mole) d'imidazo[1,5-a]pyridinecarbonitrile-1 (Y.A. Saednya, Synthesis 1983, 748) dans 300 ml d'éthanol pendant que l'on fait barboter HCl gazeux.

On évapore à sec la solution, reprend avec de l'eau, extrait avec de l'éther. On lave la phase organique avec de l'eau, du bicarbonate et de l'eau. On obtient après évaporation le produit. F = 141°C.

6.2. Hydrazide de l'acide imidazo[1,5-a]pyridine-carboxylique-1.

On utilise le mode opératoire décrit dans l'exemple 1, à partir de 10,2 g (0,05 mole) de l'ester précédent et 20 ml d'hydrazine. On obtient le produit qui fond à 189°C.

6.3. [(Nitro-5 furannyl-2)méthylène]hydrazide de l'acide imidazo[1,5-a]pyridinecarboxylique-1.

On prépare le produit selon l'exemple 1, à partir de 1,3 g (0,007 mole) de l'hydrazide précédent et de 1,1 g (0,008 mole) de nitro-5 furannecarboxaldéhyde-2. On obtient le produit qui fond avec décomposition à 300°C.

Les hydrazides (II), produits de départ, sont représentés dans le tableau I.

Les composés finals (I) préparés à titre d'exemples sont représentés dans le tableau II.

TABLEAU I

R - CO - NH - $NH_2$ (II)

| R | F(°C) | R | F(°C) |
|---|---|---|---|
| 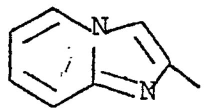 | 195-198 | 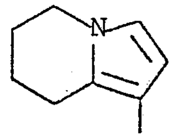 | 142 |
| 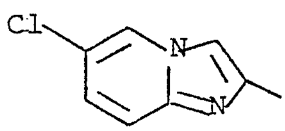 | 237 | 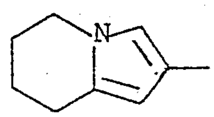 | 127 |
| 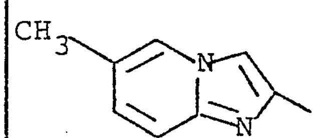 | 207 | 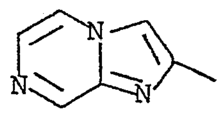 | 269 |
| 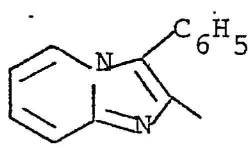 | 233 | 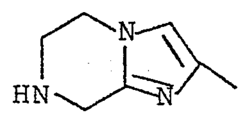 | 179 |
| 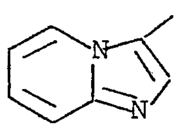 | 224 | 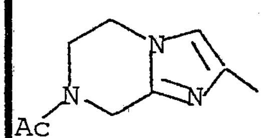 | 182 |
| 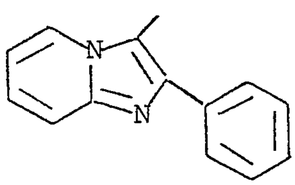 | 192 | 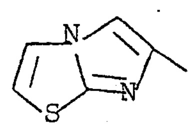 | 230 |

TABLEAU I suite

| R | F(°C) | R | F(°C) |
|---|---|---|---|
| 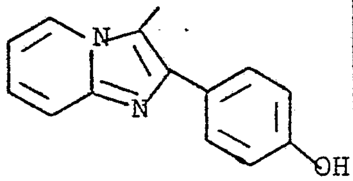 | 258 | 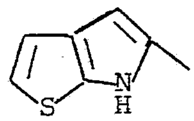 | 234 |
| 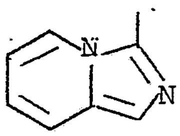 | 159 | 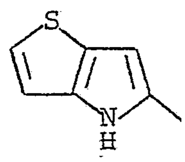 | 270 |
| 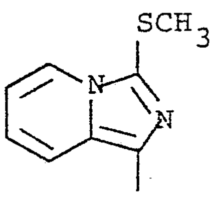 | 156 | 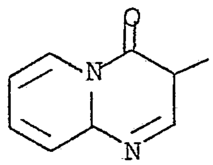 | 218-220 |
| 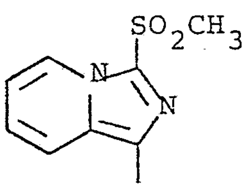 | 226 | 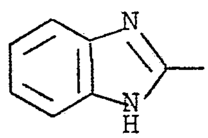 | 243 |
| 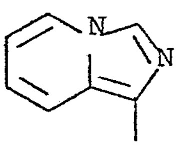 | 189 | 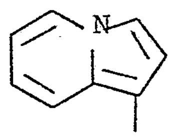 | 130 |
| 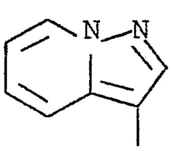 | 195 | 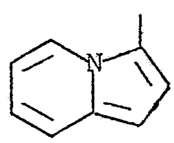 | 172 |

TABLEAU II

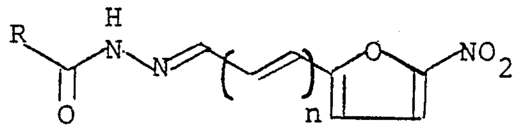

(I)

| Composé | R | n | F(°C) |
|---|---|---|---|
| 1 | 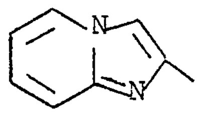 | 0 | 280 |
| 2 | 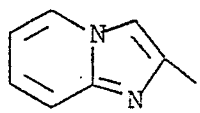 | 1 | 310 |
| 3 | 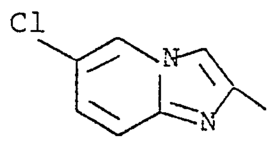 | 0 | 316 |
| 4 | 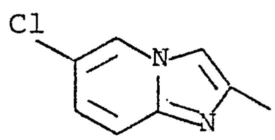 | 1 | 318 |
| 5 | 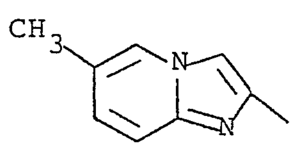 | 0 | 282 |
| 6 | 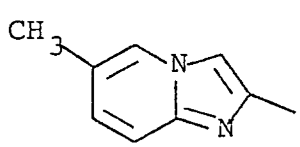 | 1 | 302 |
| 7 | 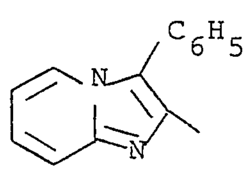 | 0 | 245 |

TABLEAU II (suite 1)

| Composé | R | n | F(°C) |
|---|---|---|---|
| 8 | 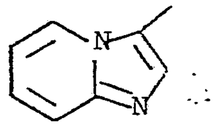 | 0 | 260 |
| 9 | 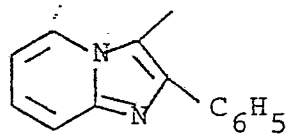 | 0 | 218 |
| 10 | 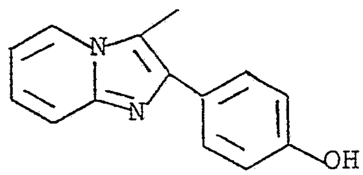 | 0 | 244-245 |
| 11 | 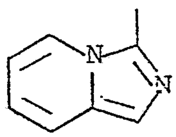 | 0 | 300 |
| 12 | 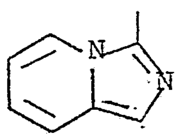 | 1 | 255-256 |
| 13 | 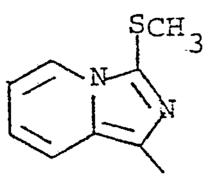 | 0 | 258 |
| 14 | 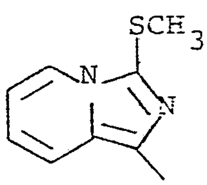 | 1 | 250 |
| 15 | 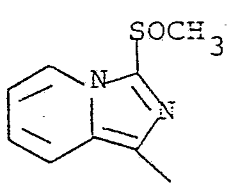 | 0 | 294 |

TABLEAU II (suite 2)

| Composé | R | n | F(°C) |
|---|---|---|---|
| 16 | 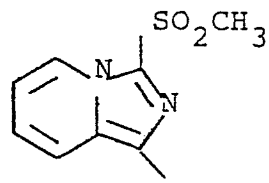 | 0 | 305-306 |
| 17 | 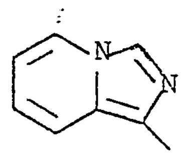 | 0 | 300 |
| 18 | 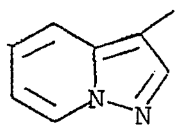 | 0 | 264 |
| 19 | 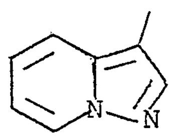 | 1 | 250 |
| 20 | 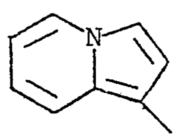 | 0 | 236 |
| 21 | 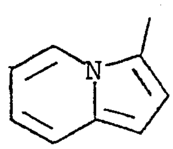 | 0 | 218 |
| 22 | 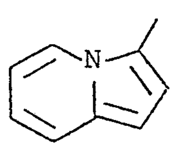 | 1 | 211 |
| 23 | 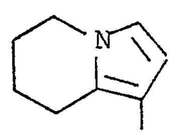 | 0 | 146 |

TABLEAU II (suite 3)

| Composé | R | n | F(°C) |
|---|---|---|---|
| 24 | 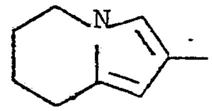 | 0 | 242 |
| 25 | 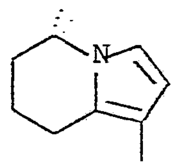 | 1 | 219 |
| 26 | 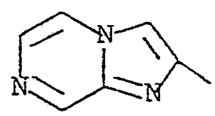 | 0 | 329-330 |
| 27 | 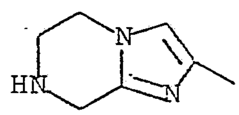 | 0 | 242 |
| 28 | 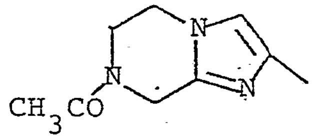 | 0 | 255 |
| 29 | 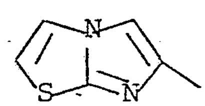 | 0 | 316 |
| 30 | 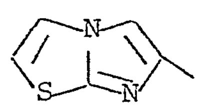 | 1 | 332 |
| 31 | 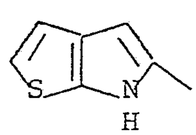 | 0 | 272 |

TABLEAU II (suite 4)

| Composé | R | n | F(°C) |
|---|---|---|---|
| 32 | 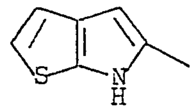 | 1 | 271 |
| 33 | 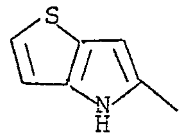 | 0 | 269 |
| 34 | 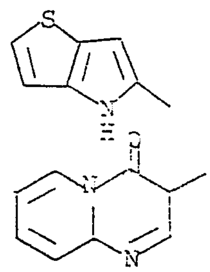 | 1 | 266 |
| 35 | | 0 | 315 |
| 36 | 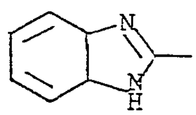 | 0 | 289 |

Les composés de l'invention ont été soumis à des essais pharmacologiques dans le domaine antibactérien, antiparasitaire et antifongique.

Les composés de l'invention présentent une activité inhibitrice "in vitro" et "in vivo" vis-à-vis d'un grand nombre de souches comprenant notamment : Staphylococcus aureus, Escherichia Coli, Mycobacterium ranae, Pseudomonas aeruginosa, Proteus Vulgaris, Vibrio Cholerae, Klebsiella pneumoniae, Trichomonas, Salmonella, Shighella Flexneri, Candida Albicans, qui permettent d'envisager leur utilisation dans diverses infections bactériennes et parasitaires, notamment au niveau intestinal.

La concentration minimale inhibitrice "in vitro" déterminée après mise en solution des composés dans le diméthylformamide (0,1 %) varie suivant les souches de 0,05 µg/ml à 20 µg/ml.

Les études réalisées "in vivo" sur l'infection expérimentale chez la souris démontrent l'activité orale des composés puisque ceux-ci inhibent la mortalité induite par plusieurs souches de bactéries et provoquent une stérilisation complète du tube digestif de la souris.

Les composés présentent une très faible toxicité qui est en général très supérieure à 1 g/kg.

Les composés de l'invention peuvent être utilisés en clinique chez l'homme à des doses de 20 mg à 1 g/jour, la posologie unitaire étant comprise entre 5 et 200 mg ; les composés peuvent être utilisés chez l'animal à des doses de 1 à 20 mg/kg/jour.

Les composés peuvent être présentés sous toute forme appropriée pour l'administration par voie orale, rectale ou parentérale, par exemple sous la forme de capsules, comprimés, granulés, gélules ou solutés liquides, sirops ou suspension buvables, et contenir les excipients appropriés.

Les composés de l'invention peuvent être utilisés chez l'animal et l'homme comme antibactériens, antiseptiques intestinaux, antifongiques et/ou antiprotozoaires. Au niveau intestinal chez l'homme, ils pourront être employés pour soigner les colopathies fonctionnelles infectieuses, les diarrhées d'origine alimentaire ou non, les entérites, les enterocolites, les dysenteries bacillaires.

Les composés de l'invention peuvent être également utilisés pour la protection et la conservation des aliments et pour favoriser la croissance du bétail, en luttant contre les infections bactériennes et parasitaires.

## Revendications

1. Dérivés du nitrofuranne répondant à la formule (I)

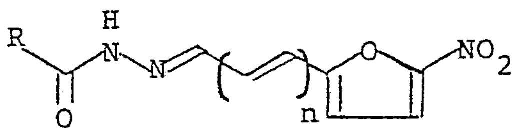

(I)

dans laquelle n est 0 ou 1 et R est un radical imidazo[1,2-a]pyridinyle-2, imidazo[2,1-b]thiazolyle-6, imidazo[1,2-a]pyrazinyle-2, tétrahydro-5,6,7,8 imidazo[1,2-a]pyrazinyle-2, acétyl-7 tétrahydro-5,6,7,8 imidazo[1,2-a]pyrazinyle-2, imidazo[1,5-a]pyridinyle-1, imidazo[1,5-a]pyridinyle-3, pyrazolo[1,5-a]pyridinyle-3, indolizinyle-1, indolizinyle-3, tétrahydro-5,6,7,8 indolizinyle-1, tétrahydro-5,6,7,8 indolizinyle-2, 6H-thieno[2,3-b]pyrrolyle-5, 4H-thiéno[3,2-b]pyrrolyle-5, oxo-4 4H-pyrido[1,2-a]pyrimidinyle-3, 2H-benzimidazolyle-2, imidazo[1,2-a]pyridinyle-3.

2. Procédé de préparation des composés selon la revendication 1, procédé caractérisé en ce que l'on condense un hydrazide de formule (II)

$R - CO - NH - NH_2$ (II)

avec le nitro-5 furannecarboxaldéhyde-2 ou le (nitro-5 furyl-2)-3 propène-2al, dans un solvant alcoolique, à une température allant de 20° C à la température de reflux du solvant.

3. Médicament caractérisé en ce qu'il contient un composé tel que spécifié dans la revendication 1.

4. Composition pharmaceutique caractérisée en ce qu'elle contient un composé tel que spécifié dans la revendication 1 en association avec tout excipient approprié.

Revendication pour les Etats contractants suivants: AT, ES, GR

Procédé de préparation des dérivés du nitrofuranne répondant à la formule (I)

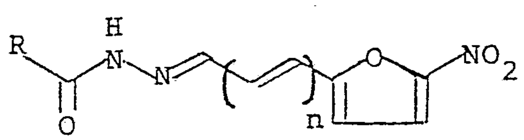

(I)

dans laquelle n est 0 ou 1 et R est un radical imidazo[1,2-a]pyridinyle-2, imidazo[2,1-b]thiazolyle-6, imidazo[1,2-a]pyrazinyle-2, tétrahydro-5,6,7,8 imidazo[1,2-a]pyrazinyle-2, acétyl-7 tétrahydro-5,6,7,8 imidazo[1,2-a]pyrazinyle-2, imidazo[1,5-a]pyridinyle-1, imidazo[1,5-a]pyridinyle-3, pyrazolo[1,5-a]pyridinyle-3, indolizinyle-1, indolizinyle-3, tétrahydro-5,6,7,8 indolizinyle-1, tétrahydro-5,6,7,8 indolizinyle-2, 6H-thieno[2,3-b]pyrrolyle-5, 4H-thiéno[3,2-b]pyrrolyle-5, oxo-4 4H-pyrido[1,2-a]pyrimidinyle-3, 2H-benzimidazolyle-2, imidazo[1,2-a]pyridinyle-3,
procédé caractérisé en ce que l'on condense un hydrazide de formule (II)

R - CO - NH - $NH_2$ (II)

avec le nitro-5 furannecarboxaldéhyde-2 ou le (nitro-5 furyl-2)-3 propène-2al, dans un solvant alcoolique, à une température allant de 20° C à la température de reflux du solvant.